# EUROPEAN PATENT APPLICATION

(11) **EP 4 492 445 A1**
(43) Date of publication of application: **15.01.2025**
(21) Application number: 22933671.4
(22) Date of filing: 20.12.2022
(51) Int. Cl.: H01L 23/00, H01L 21/02, H01L 21/50

(54) **MATERIAL SELECTION ASSISTANCE DEVICE, METHOD, AND PROGRAM**

(30) Priority: 24.03.2022 WO PCT/JP2022/014169
(71) Applicant: Resonac Corporation, Tokyo 105-7325 (JP)
(72) Inventor: ABE, Keita, Tokyo 100-6606 (JP); UEDA, Atsuko, Tokyo 100-6606 (JP); NAKATA, Mitsuki, Tokyo 100-6606 (JP)
(74) Representative: Hoffmann Eitle
(86) International application number: PCT/JP2022/046968
(87) International publication number: WO 2023/181543

(57) **Abstract**

A reception unit (32) receives shape values pertaining to the shapes of respective configurations of a semiconductor package, and physical property values of materials to use. Each time the shape values and the physical property values are received, a simulation unit 34 simulates warpage of a substrate on the basis of the received shape values and physical property values. A calculation unit (36) calculates, with regard to each of plural materials registered in a material database (40) in advance, the difference between physical property values of the plural materials and the received physical property value. A display control unit (38) displays simulation results and the results of calculating the difference in physical property values at a display device.

## Description

### Technical Field

The present disclosure relates to a material selection assistance apparatus, a material selection assistance method, and a material selection assistance program.

### Background Art

Conventionally, a material to be used for manufacturing a product is selected in consideration of phenomena occurring in the product according to design of the product and physical properties of the used material.

For example, as a method of selecting materials of a panel used for manufacturing a semiconductor package, a method of selecting materials that enables a panel having a sufficiently small amount of warpage to be manufactured is proposed (see Japanese Patent Application Laid-Open (JP-A) No. 2020-38924). The method described in JP-A No. 2020-38924 relates to the method of selecting materials of a panel including a back surface coating layer, a large number of semiconductor elements, a sealing layer, and an insulating layer. In this method, a virtual model of a panel to which characteristics of materials of the back surface coating layer, the sealing layer, and the insulating layer are input is built using structural analysis software. In addition, in this method, the amount of warpage of the virtual model is calculated, and the characteristics of the materials affecting the amount of warpage of the panel, from the materials of the back surface coating layer, the sealing layer, and the insulating layer, are ascertained by structural analysis. In addition, in this method, at least one of the materials of the back surface coating layer, the sealing layer, and the insulating layer is newly selected on the basis of the ascertained information so that the amount of warpage of the virtual model is reduced.

### Citation List

### Patent Literature

Patent Literature 1: Japanese Patent Application Laid-Open (JP-A) No. 2020-38924

### SUMMARY OF INVENTION

### Technical Problem

As a process of selecting a material for general product manufacturing, the following process is assumed. First, product design information is transmitted from a customer on a product manufacturing side to a person in charge on a material providing side. Then, on the material providing side, a phenomenon of the product (for example, warpage of a substrate in a semiconductor package) in a case where a material selected on the material providing side is applied to the product is simulated, and a simulation result is reported to the customer. Here, in a case where approval from the customer is not obtained, design information, a material, or the like is changed. On the material providing side, a simulation is performed again on the basis of this changed design information and material, and a result thereof is reported to the customer. From the customer side, a problem arises in that it is not desired to provide detailed design information at a product design stage, the above process is repeatedly performed, and it takes time to perform communication with regard to a selected material in some cases until approval from the customer is obtained.

The disclosure has been made in view of the above described problem, and an object thereof is to provide a material selection assistance apparatus, method, and program enabling communication for material selection in product manufacturing to be reduced.

### Solution to Problem

In order to achieve the above-described object, there is provided a material selection assistance apparatus according to the disclosure including: a reception unit that receives a shape value pertaining to a shape of a product and a physical property value of a material used for the product; a simulation unit that simulates a phenomenon occurring in the product based on the shape value and the physical property value received, each time the shape value and the physical property value are received by the reception unit; a calculation unit that calculates, with regard to each of a plurality of materials registered in advance, a difference between a physical property value of each of the plurality of materials and the physical property value received by the reception unit; and a display control unit that performs control to display a simulation result obtained by the simulation unit and the difference calculated by the calculation unit at a display device. This enables communication for material selection in product manufacturing to be reduced.

The simulation unit may simulate the phenomenon using a function created in advance to output a physical quantity indicating the phenomenon, with the shape value, the physical property value, and a simulation condition as inputs. This enables the simulation to be easily performed, and enables output values of consecutive simulations with consecutively changed input values to be quickly obtained.

The display control unit may perform control to display, on one screen, an input region to which the shape value and the physical property value are input, a display region of the simulation result, and a display region of the difference with regard to each of the plurality of materials. This enables the input value and the physical property value to be easily adjusted while the simulation result and a calculation result of the difference between the physical property values are checked.

The display control unit may perform control to display the difference with regard to each of the plurality of materials in a graph. This enables the difference between the physical property values for each material to be intuitively and easily checked.

The reception unit may receive a display instruction for the difference with regard to each of the plurality of materials, and when the display instruction is received, the calculation unit may calculate a difference between the shape value and the physical property value received by the reception unit, and a physical property value of each of the plurality of materials. This enables the difference between the physical property values to be calculated only when necessary.

The reception unit may receive a condition for one or more characteristics required of the product other than the phenomenon, the material selection assistance apparatus may include a filter unit that performs filtering of each of the plurality of materials based on whether or not the condition received by the reception unit is satisfied, and the display control unit may perform control to display a filtering result obtained by the filter unit at the display device in association with the display of the difference with regard to each of the plurality of materials. This enables material selection to be more easily performed in consideration of other characteristics as well as the simulation results and the difference between the physical property values.

The display control unit may superimpose and display a mask indicating that the condition is not satisfied on a graph for a material determined by the filter unit to be a material which does not satisfy the condition among graphs respectively indicating the differences for the plurality of materials. This results in easier focus on the material that satisfies the conditions.

The material selection assistance apparatus may include a scoring unit that calculates, with regard to each of the plurality of materials, a combined score obtained by combining a score corresponding to the simulation result obtained by the simulation unit, a score corresponding to the difference, and a score determined in advance with regard to each of one or more characteristics required of the product other than the phenomenon and the difference, wherein the display control unit may perform control to display the combined score calculated by the scoring unit at the display device with regard to each of the plurality of materials. This enables the material selection to be more easily performed in the comprehensive consideration of demanded characteristics.

The reception unit may receive a selection of a target from among the simulation result, the difference, and the one or more characteristics reflected in the combined score, and a specific weighting when scores for the selected target are combined into the combined score, and the scoring unit may calculate the combined score based on the target and the specific weighting received by the reception unit. This enables the selection of the material to be more easily performed on the basis of the score obtained by reflecting which characteristic is more emphasized.

The display control unit may display details of a score with regard to each of characteristics of the combined score. This enables the material selection to be determined in consideration of the details of the combined score.

In a case in which the product is a semiconductor package, the phenomenon may be warpage of the product, stress of the product, or a service life of solder in the product.

In addition, there is provided a material selection assistance method according to the disclosure including: receiving, by a reception unit, a shape value pertaining to a shape of a product and a physical property value of a material used for the product; simulating, by a simulation unit, a phenomenon occurring in the product, based on the shape value and the physical property value received each time the shape value and the physical property value are received by the reception unit; calculating, by a calculation unit, with regard to each of a plurality of materials registered in advance, a difference between a physical property value of each of the plurality of materials and the physical property value received by the reception unit; and performing control, by a display control unit, to display a simulation result obtained by the simulation unit and the difference calculated by the calculation unit at a display device.

In addition, there is provided a material selection assistance program according to the disclosure for causing a computer to function as: a reception unit that receives a shape value pertaining to a shape of a product and a physical property value of a material used for the product; a simulation unit that simulates a phenomenon occurring in the product based on the shape value and the physical property value received, each time the shape value and the physical property value are received by the reception unit; a calculation unit that calculates, with regard to each of a plurality of materials registered in advance, a difference between a physical property value of each of the plurality of materials and the physical property value received by the reception unit; and a display control unit that performs control to display a simulation result obtained by the simulation unit and the difference calculated by the calculation unit at a display device.

### Advantageous Effects of Invention

The material selection assistance apparatus, method, and program according to the disclosure enable communication for the material selection in product manufacturing to be reduced.

### BRIEF DESCRIPTION OF DRAWINGS

Fig. 1 is a block diagram illustrating a hardware configuration of a material selection assistance apparatus according to first and second embodiments.
Fig. 2 is a block diagram illustrating an example of a functional configuration of the material selection assistance apparatus according to the first embodiment.
Fig. 3 is a view illustrating an example of a display screen according to the first embodiment.
Fig. 4 is a table illustrating an example of a material DB.
Fig. 5 is a view illustrating an example of the display screen according to the first embodiment.
Fig. 6 is a view illustrating an example of the display screen according to the first embodiment.
Fig. 7 is a flowchart illustrating a flow of a material selection assistance process according to the first embodiment.
Fig. 8 is a block diagram illustrating an example of a functional configuration of the material selection assistance apparatus according to the second embodiment.
Fig. 9 is a view illustrating an example of a display screen according to the second embodiment.
Fig. 10 is a table illustrating an example of a score table.
Fig. 11 is a diagram illustrating an example of a display of a filter designation screen and a filtering result.
Fig. 12 is a diagram illustrating an example of a display of a score designation screen and a combined score.
Fig. 13 is a diagram illustrating an example of a display of a filter designation screen and a filtering result.
Fig. 14 is a diagram illustrating an example of a display of a score designation screen and a combined score.
Fig. 15 is a flowchart illustrating a flow of a material selection assistance process according to the second embodiment.
Fig. 16 is a block diagram illustrating a schematic configuration of a material selection assistance system according to Modification Example 2.

### DESCRIPTION OF EMBODIMENTS

Hereinafter, an example of the present embodiment will be described with reference to the drawings.

### <First Embodiment>

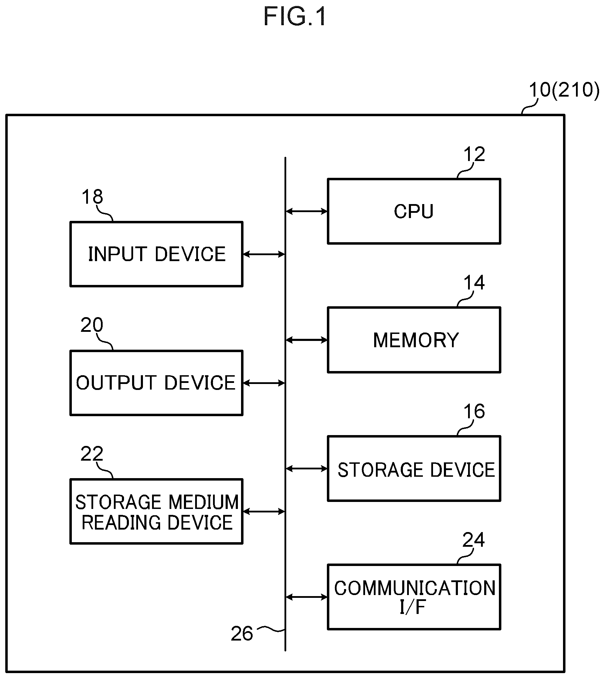

Fig. 1 is a block diagram illustrating a hardware configuration of a material selection assistance apparatus 10 according to a first embodiment. As illustrated in Fig. 1, the material selection assistance apparatus 10 includes a central processing unit (CPU) 12, a memory 14, a storage device 16, an input device 18, an output device 20, a storage medium reading device 22, and a communication interface (I/F) 24. Configurations thereof are communicatively connected to each other via a bus 26.

The storage device 16 stores a material selection assistance program for executing a material selection assistance process to be described below. The CPU 12 is a central processing unit, and executes various programs or controls the configurations. That is, the CPU 12 reads a program from the storage device 16, and executes the program using the memory 14 as a work area. The CPU 12 performs control of each of the above-described configurations and various types of arithmetic processing in accordance with the programs stored in the storage device 16.

The memory 14 includes a random access memory (RAM), and as a work area, temporarily stores a program and data. The storage device 16 includes a read only memory (ROM), a hard disk drive (HDD), a solid state drive (SSD), and the like, and stores various programs including an operating system and various items of data.

The input device 18 is a device for performing various inputs, such as a keyboard or a mouse. The output device 20 is, for example, a device for outputting various items of information, such as a display or a printer. A touch panel display may be employed as the output device 20, and may function as the input device 18.

The storage medium reading device 22 performs functions of reading data stored in various storage mediums such as a compact disc (CD)-ROM, a digital versatile disc (DVD)-ROM, a Blu-ray disc, and a universal serial bus (USB) memory, writing data to the storage medium, and the like. The communication I/F24 is an interface for communicating with other instruments, and a standard such as Ethernet (registered trademark), FDDI, or Wi-Fi (registered trademark) is used.

Next, functional configurations of the material selection assistance apparatus 10 according to the first embodiment will be described. In the following embodiment, a case in which a target product is a semiconductor package and warpage of a semiconductor package substrate is simulated as a phenomenon occurring in the product will be described as an example.

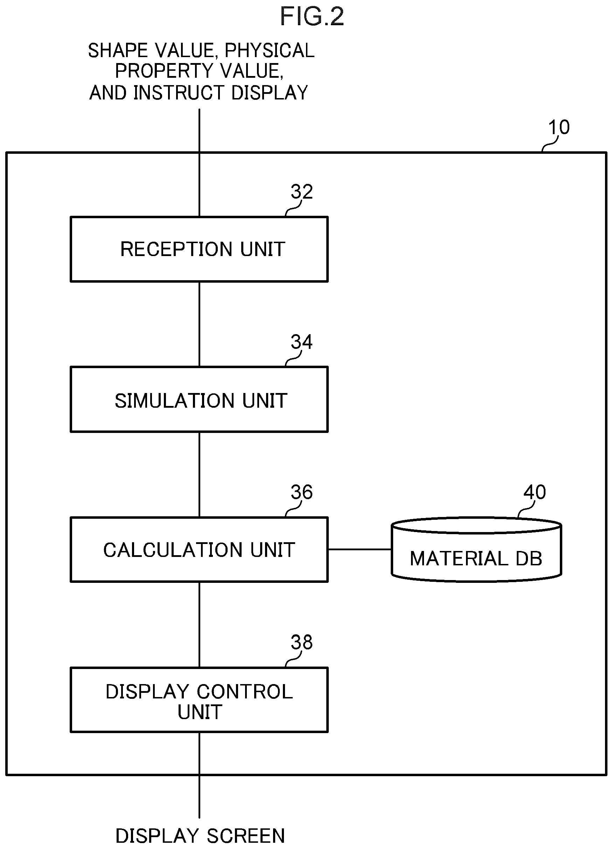

Fig. 2 is a block diagram illustrating an example of the functional configurations of the material selection assistance apparatus 10. As illustrated in Fig. 2, the material selection assistance apparatus 10 includes a reception unit 32, a simulation unit 34, a calculation unit 36, and a display control unit 38 as the functional configurations. A material database (DB) 40 is stored in a predetermined storage area of the material selection assistance apparatus 10. The functional configurations are realized by causing the CPU 12 to read the material selection assistance program stored in the storage device 16, load the program in the memory 14, and execute the program.

The reception unit 32 receives a shape value pertaining to a shape of a product and a physical property value of a material used for the product. In the present embodiment, the shape value pertaining to the shape is a value indicating a geometric shape such as a dimension or an arrangement of each configuration of the semiconductor package. Each configuration of the semiconductor package may be a core material, a build-up material, a chip, a capillary underfill, copper, or a solder resist. The configuration may include a stiffener and a through-hole. There is no need to include all these configurations, and some thereof may be omitted or added according to an actual configuration. A dimension of each configuration is a size of a length, a width, or a thickness, and the arrangement is a layered positional relationship or the like between the configurations.

The physical property values of a material are values of a glass transition temperature, the Young's modulus, and a coefficient of thermal expansion (linear expansion coefficient) with regard to each configuration of the semiconductor package. However, some physical property values have a plurality of values depending on a condition of a thermal stress field at the time of measurement and simulation, such as a temperature and a strain rate. Therefore, two or more values in any conditions may be included as respective physical property values. Depending on a method of computing warpage by the simulation unit 34 to be described below, a physical property value that is not actually acquired or does not exist may be input.

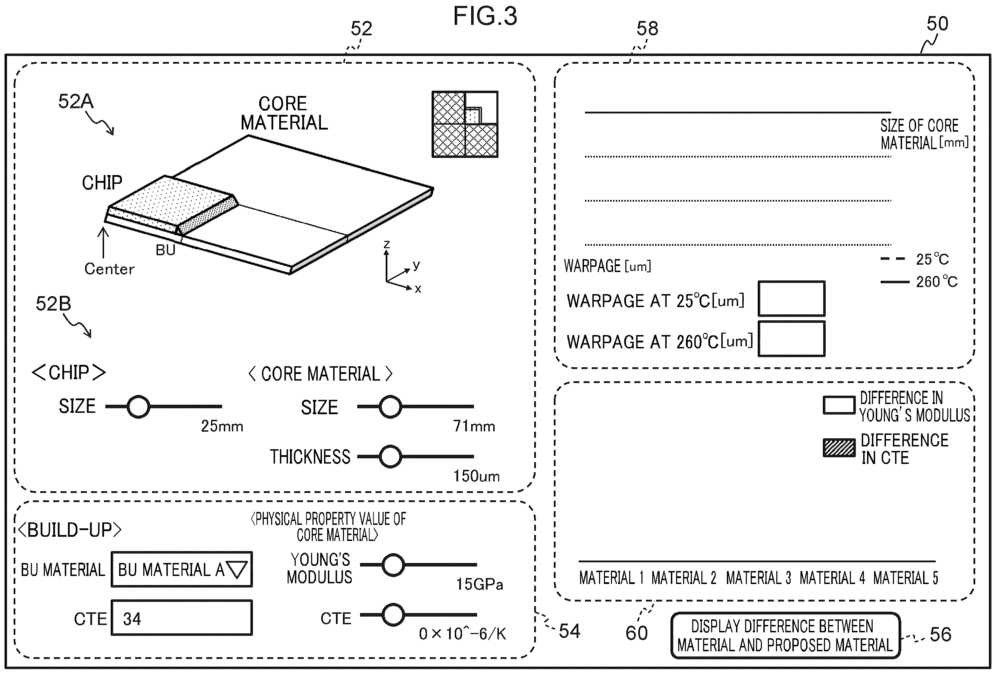

Specifically, the reception unit 32 receives the shape value and the physical property value input on the display screen 50 as illustrated in Fig. 3, for example. The display screen 50 is displayed on a display which is an example of the output device 20 by a display control unit 38 to be described below. In the example of Fig. 3, the display screen 50 includes a shape value input region 52, a physical property value input region 54, a display instruction button 56, a simulation result display region 58, and a physical property value difference display region 60.

The shape value input region 52 further includes an arrangement designation region 52A and a dimension input region 52B. In the arrangement designation region 52A, for example, an image diagram of each configuration of the semiconductor package is displayed, and an arrangement of each configuration can be changed by a drag operation or the like. In the dimension input region 52B, for example, sizes (vertical dimension × horizontal dimension) and a thickness of each configuration can be input by sliding a slide bar. The input of the dimension is not limited to the input using the slide bar, and may be an input performed by using a text box for directly inputting a numerical value, a pull-down menu for selecting a desired size from options, or the like.

The physical property value input region 54 includes a text box, a slide bar, or the like for inputting the physical property value of each configuration. The example of Fig. 3 provides a configuration in which the core material is a target of material selection assistance. Regarding the core material in the configuration, the physical property value input region 54 includes a slide bar for inputting a physical property value (in the example of Fig. 3, the Young's modulus and the CTE) which is required of the core material. In the example of Fig. 3, the physical property value input region 54 includes a pull-down menu for selecting a build-up material, and a text box for inputting a coefficient of thermal expansion (CTE) of the selected build-up material, regarding an input of a physical property value of build-up (BU) in the configurations.

The reception unit 32 receives, via the display screen 50, the shape value input in the shape value input region 52 and the physical property value input in the physical property value input region 54.

The reception unit 32 receives a display instruction of a difference (details thereof will be described below) between the physical property value of each of the plurality of materials registered in advance and the input physical property value. Specifically, in a case in which the display instruction button 56 is selected on the display screen 50, the reception unit 32 receives a display instruction.

In the example of the display screen 50 illustrated in Fig. 3, only some of the individual configurations, shape values, and physical property values of the semiconductor package are provided for convenience of drawing. The simulation result display region 58 and the physical property value difference display region 60 will be described below.

The simulation unit 34 simulates the amount of warpage occurring in the semiconductor package substrate on the basis of the shape values and the physical property values received by the reception unit 32. The simulation unit 34 executes a simulation of warpage each time the input of the shape value and the physical property value is changed, that is, each time the reception unit 32 receives a new shape value and a new physical property value. Specifically, the simulation unit 34 simulates the warpage by using a function created in advance to output the amount of warpage, with the shape value, the physical property value, and a simulation condition as inputs.

More specifically, since the warpage is a problem caused by thermal deformation of the semiconductor package due to a temperature change, the simulation unit 34 sets an initial temperature, a final temperature, and a profile of a temporal change in temperature as simulation conditions, and performs thermal stress analysis. The simulation unit 34 simulates, as the amount of warpage, the amount of displacement of an end point of the substrate (core material) in a vertical (Z-axis) direction. In the case of the warpage having a complicated W shape, a complicated M shape, or the like, a simulation may be performed using an in-plane (X-Y plane) distribution of the amount of displacement in the vertical (Z-axis) direction and amounts of displacement in both axis (X-axis and Y-axis) directions in the plane as output values. The simulation unit 34 may simulate the amount of warpage in the state of the final temperature, or may acquire the history of warpage in the middle of a temperature change.

Here, the simulation of warpage is conceivably performed by dividing differential equations for thermal stress analysis into, for example, finite element meshes and solving a number of simultaneous algebraic equations corresponding to the number of meshes. However, in this case, it takes time to obtain a simulation result. For example, six hours of a computing time may be taken for simulating one case of warpage. In this respect, a function for outputting the warpage corresponding to a median of the input shape value and the input physical property value at high speed is created in advance. As a method of creating this function, for example, multiple regression analysis including terms up to the squared term of both the input values (the shape value and the physical property value) may be employed, or another method of creating a function may be employed.

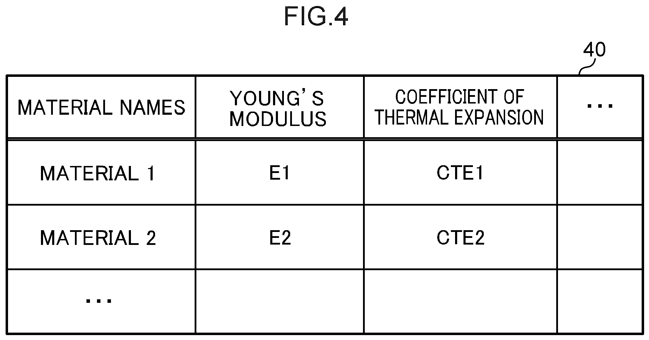

The calculation unit 36 calculates, with regard to each of a plurality of materials registered in the material DB 40 in advance, a difference between the physical property value received by the reception unit 32 and a physical property value of each of the plurality of materials. Fig. 4 illustrates an example of the material DB 40. In the example of Fig. 4, with regard to each material, a "material name" of the material and physical property values such as "Young's modulus" and the "coefficient of thermal expansion" are stored in association with each other in the material DB 40. Instead of or in addition to the material name, a product model name and a product model number of the material, a unique identification number, or the like may be used. Specifically, the calculation unit 36 reads the physical property value of each material from the material DB 40, and calculates, for each type of physical property value, a difference between the physical property value read from the material DB and a physical property value received by the reception unit 32.

In a case in which a display instruction of the physical property value difference is received by the reception unit 32, that is, in a case in which the display instruction button 56 is selected on the display screen 50, the calculation unit 36 may calculate a difference between the physical property value read from the material DB and a physical property value input to the physical property value input region 54.

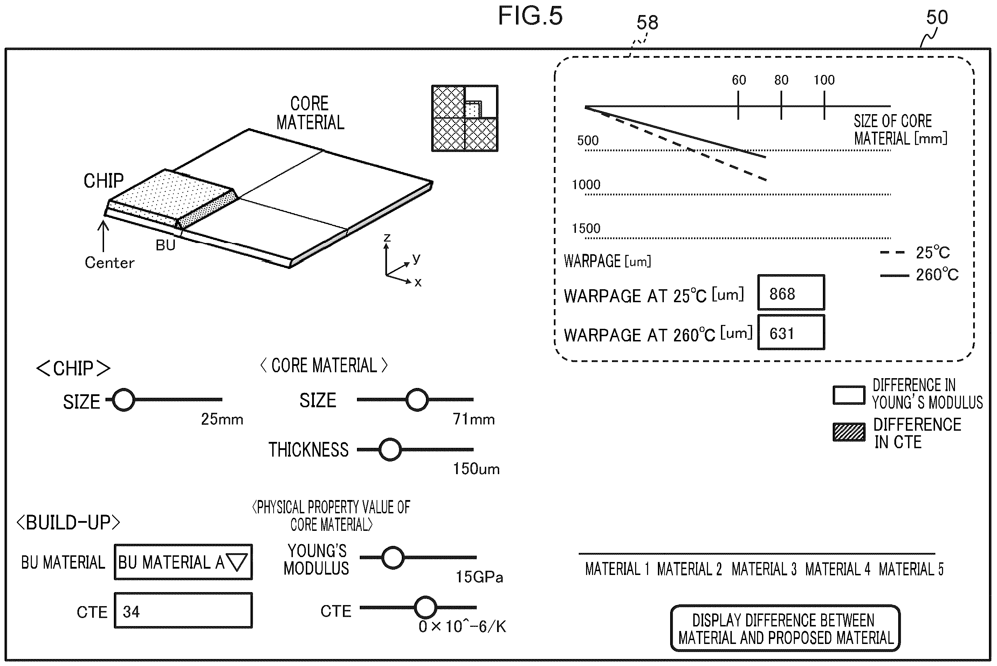

The display control unit 38 performs control to display the display screen 50 on the display. The display control unit 38 displays a simulation result obtained by the simulation unit 34 in the simulation result display region 58 of the display screen 50. Fig. 5 illustrates an example of the display screen 50 on which a simulation result is displayed in the simulation result display region 58. In the example of Fig. 5, the amount of warpage with respect to the size (length × width) of the core material is illustrated on a graph with respect to two degrees of temperatures. The amount of warpage with respect to the size of the core material input in the shape value input region 52 is displayed in a text box.

As described above, the simulation unit 34 executes a simulation of warpage each time the shape value and the physical property value are changed. Hence, the display control unit 38 updates the display in the simulation result display region 58 each time the shape value and the physical property value are changed.

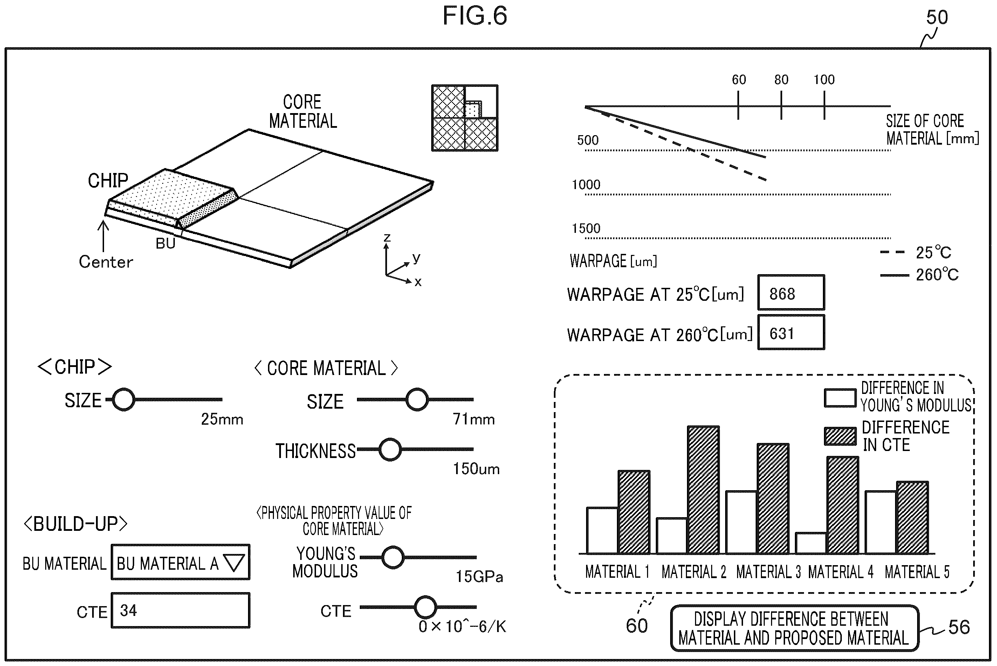

The display control unit 38 displays the difference between the physical property values calculated by the calculation unit 36 in the physical property value difference display region 60. As illustrated in Fig. 6, the display control unit 38 may display the difference between the physical property values on a graph. Fig. 6 illustrates an example in which the difference between the physical property values is represented by a bar graph. The display control unit 38 may display a number indicating the difference between the physical property values together with the graph.

As described above, in the case in which the calculation unit 36 calculates the difference between the physical property values when the display instruction button 56 is selected, the display control unit 38 updates the display of the physical property value difference display region 60 each time the display instruction button 56 is selected.

Next, operations of the material selection assistance apparatus 10 according to the first embodiment will be described.

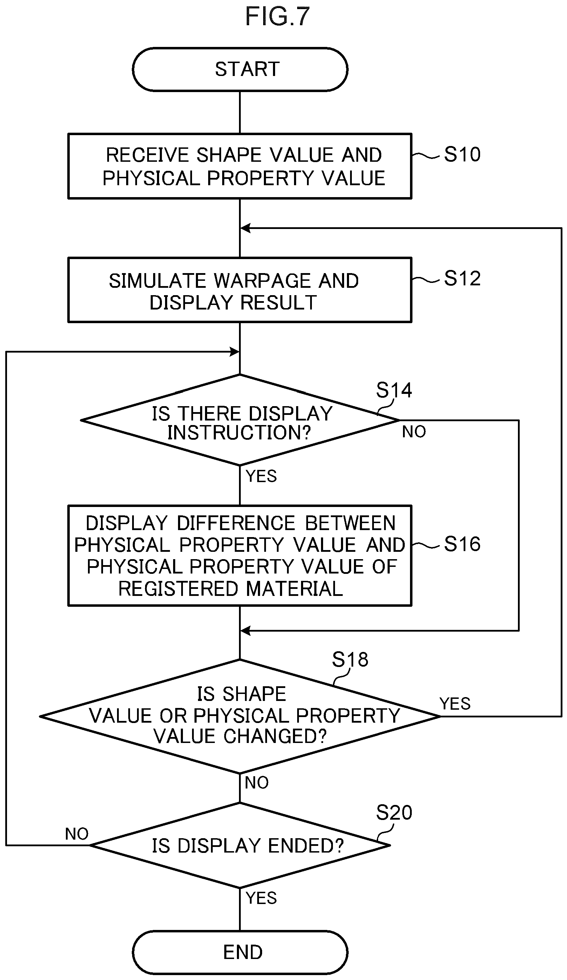

Fig. 7 is a flowchart illustrating a flow of a material selection assistance process executed by the CPU 12 of the material selection assistance apparatus 10. When the CPU 12 reads the material selection assistance program from the storage device 16, loads the program in the memory 14, and executes the program, the CPU 12 functions as the functional configurations of the material selection assistance apparatus 10, and the material selection assistance process illustrated in Fig. 7 is executed. The material selection assistance process is an example of the material selection assistance method of the disclosure.

In step S10, the display control unit 38 displays the display screen 50 on the display. The reception unit 32 receives, via the display screen 50, the shape value input in the shape value input region 52 of the display screen 50 and the physical property value input in the physical property value input region 54 thereof.

Next, in step S12, the simulation unit 34 simulates the amount of warpage occurring in the semiconductor package substrate on the basis of the shape value and the physical property value received in step S10. The display control unit 38 displays a simulation result obtained by the simulation unit 34 in the simulation result display region 58 of the display screen 50.

Next, in step S14, the reception unit 32 determines whether or not a display instruction is received by determining whether or not the display instruction button 56 is selected on the display screen 50. The process proceeds to step S16 in the case in which the display instruction is received, and the process proceeds to step S18 in the case in which the display instruction is not received.

In step S16, the calculation unit 36 calculates, with regard to each of the plurality of materials registered in the material DB 40 in advance, a difference between a physical property value of each of the plurality of materials and the physical property value received in step S10 or step S18 to be described below. The display control unit 38 displays the difference between the physical property values calculated by the calculation unit 36 in the physical property value difference display region 60 of the display screen 50.

Next, in step S18, the reception unit 32 determines whether or not at least one of the shape value and the physical property value is changed in the shape value input region 52 and the physical property value input region 54 of the display screen 50. In the case of a change in the values, the reception unit 32 receives the changed shape value and physical property value, and the process returns to step S 12. In the case of no change in the values, the process proceeds to step S20.

In step S20, the display control unit 38 determines whether or not to end the display on the display screen 50 by determining whether or not a command indicating an end of the display is received. The process returns to step S 14 in a case in which the display is not ended, and the material selection assistance process is ended in a case in which the display is ended.

As described above, the material selection assistance apparatus according to the first embodiment receives the shape value pertaining to the shape of the product and the physical property value of the material used for the product, simulates the phenomenon occurring in the product, such as the warpage in the substrate on the basis of the received shape value and the physical property value each time the shape value and the physical property value are received, calculates, with regard to each of the plurality of materials registered in advance, the difference between the physical property value of each of the plurality of materials and the received physical property value, and performs control to display the simulation result and the difference between the physical property values on the display device. This enables communication for material selection in product manufacturing to be reduced.

For example, when a person in charge on a material providing side and a customer on a product manufacturing side meet, it is assumed that a personal computer, a tablet terminal, a smartphone, or the like that functions as the material selection assistance apparatus is used. Specifically, the person in charge on the material providing side receives the shape value of a target product from the customer and inputs the shape value to the material selection assistance apparatus. Alternatively, the customer may directly input the shape value. The physical property value may be input by the person in charge or the customer, or an initial value set in advance may be used at the start. When the shape value and the physical property value are input, a simulation result of warpage is displayed. While viewing the simulation result, the person in charge or the customer can adjust the physical property value. The shape value can also be adjusted as long as the design can be changed. The shape value and the physical property value are adjusted, and when the amount of warpage falls within a desired range, the display instruction button is selected to display a difference between the physical property values. This enables easy selection of a material having a physical property value similar to the physical property value with which the amount of warpage falls within the desired range. The shape value and the physical property value can be adjusted even after the difference between the physical property values is confirmed.

As described above, in the above-described embodiment, the simulation is not performed using the physical property value of the material registered in advance, but the physical property value with which the amount of warpage falls within the desired range is identified, and the difference between the physical property value and the physical property value of the material registered in advance is displayed. Therefore, the person in charge on the material providing side and the customer communicate with each other via the display screen of the material selection assistance apparatus. This enables a process of listening to the design information, taking the design information back to the material providing side, performing a simulation, and reporting a simulation result to be omitted. Since the shape value and the physical property value can be easily adjusted on the display screen, it is possible to avoid repetition of the above-described process in a case in which an approval for the simulation result cannot be obtained from the customer. The customer side only needs to provide the shape value that needs to be input in the material selection assistance apparatus. Hence, even in a case in which the customer side does not want to provide detailed design information, the customer side can ascertain a tendency of warpage only by disclosing a certain degree of configuration. There is a possibility that a new request or the like of the customer can be found from communication between the person in charge and the customer via the material selection assistance apparatus, a state of adjustment of the shape value and the physical property value, and the like.

### <Second Embodiment>

Next, a second embodiment will be described. In a material selection assistance apparatus according to the second embodiment, the same reference numerals are assigned to the same configurations as those of the material selection assistance apparatus 10 according to the first embodiment, and the detailed description thereof will be omitted. Since a hardware configuration of the material selection assistance apparatus according to the second embodiment is similar to the hardware configuration of the material selection assistance apparatus 10 according to the first embodiment illustrated in Fig. 1, the description thereof will be omitted.

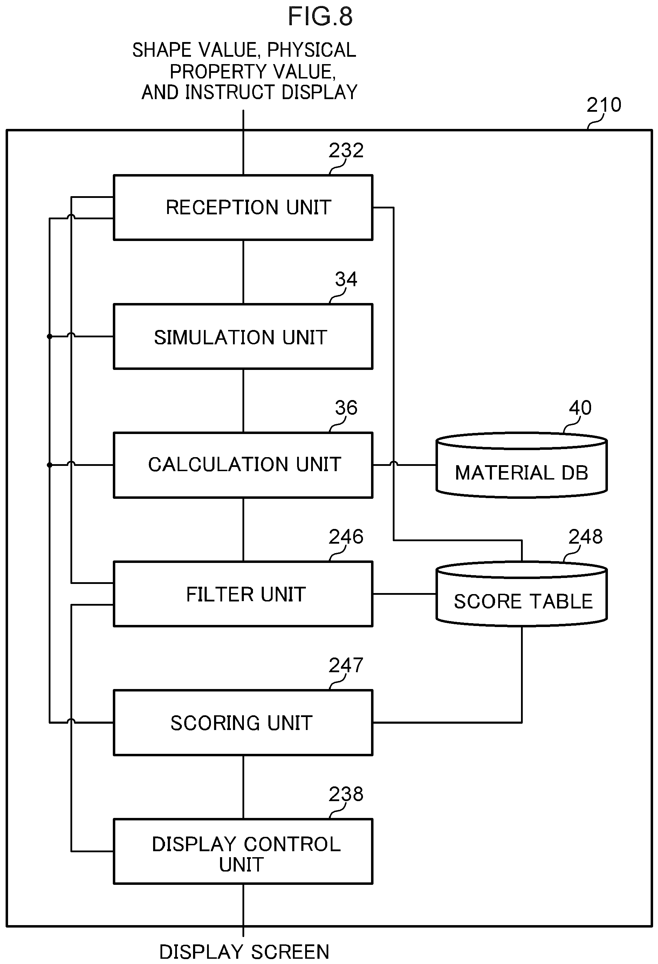

Functional configurations of the material selection assistance apparatus according to the second embodiment will be described. Fig. 8 is a block diagram illustrating an example of functional configurations of a material selection assistance apparatus 210 according to the second embodiment. As illustrated in Fig. 8, the material selection assistance apparatus 210 includes a reception unit 232, a simulation unit 34, a calculation unit 36, a filter unit 246, a scoring unit 247, and a display control unit 238 as the functional configurations. A material DB 40 and a score table 248 are stored in a predetermined storage area of the material selection assistance apparatus 210. The functional configurations are realized by causing the CPU 12 to read the material selection assistance program stored in the storage device 16, load the program in the memory 14, and execute the program.

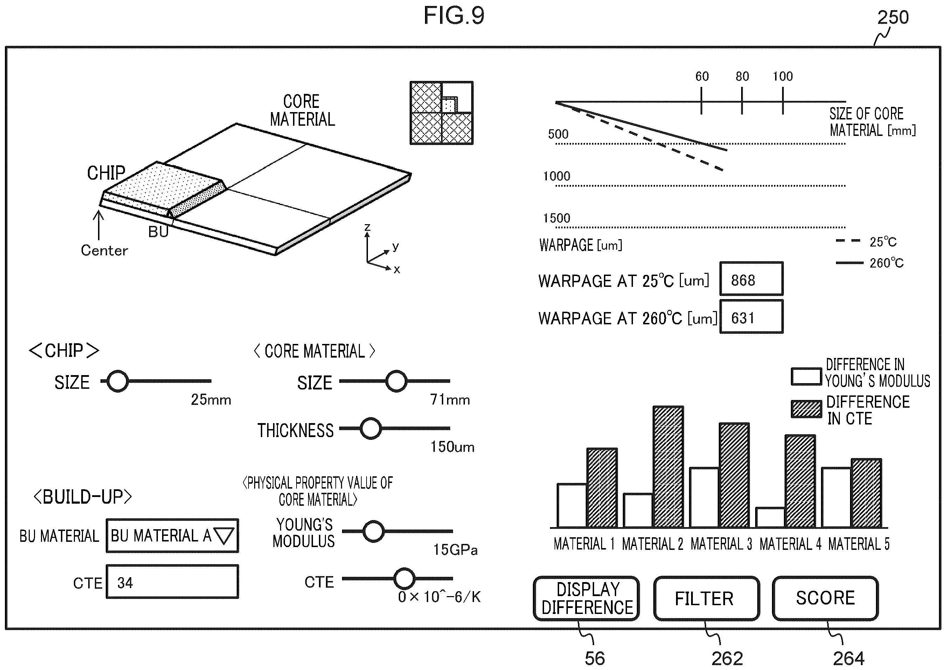

Here, Fig. 9 illustrates an example of a display screen 250 displayed by the display control unit 238 according to the second embodiment. The display screen 250 in the second embodiment includes, in addition to the display screen 50 in the first embodiment, a filter button 262 selected when filtering is executed and a score button 264 selected when a score is displayed.

Similarly to the reception unit 32 in the first embodiment, the reception unit 232 receives, via the display screen 250, a shape value input in a shape value input region 52 and a physical property value input in a physical property value input region 54.

When the filter button 262 is selected on the display screen 250, of one or more characteristics (hereinafter, referred to as "demand characteristics") required of the semiconductor package, the reception unit 232 receives a condition for a demand characteristic other than the warpage of the substrate and the difference between the physical property values. Specifically, the reception unit 232 displays a filter designation screen for designating a filtering condition with regard to each of a plurality of demand characteristics defined in the score table 248.

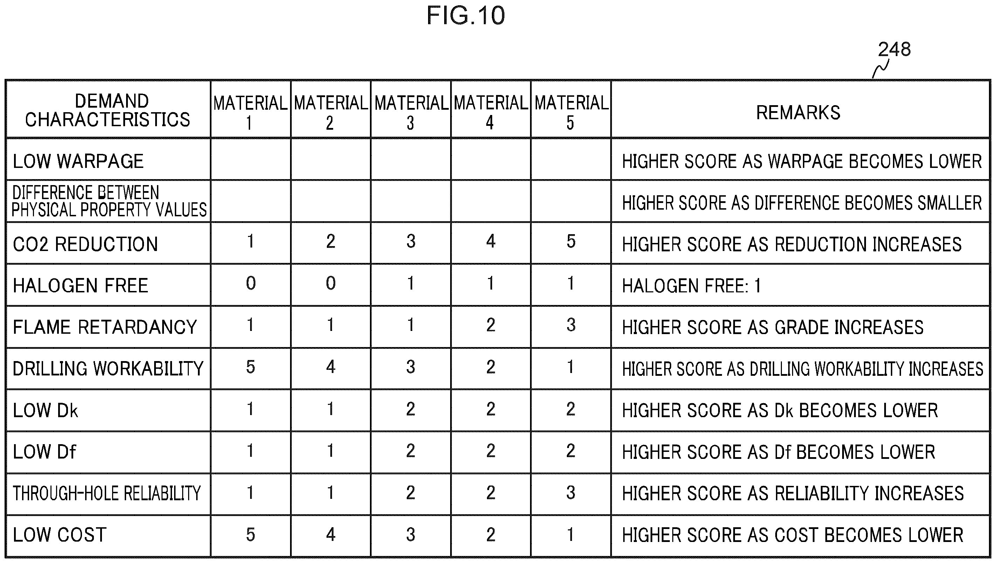

Fig. 10 illustrates an example of the score table 248. In the score table 248, scores pertaining to respective demand characteristics such as a CO2 reduction, halogen free, and flame retardancy are defined for materials (in the example of Fig. 10, materials 1 to 5), respectively. The scores of the respective demand characteristics are determined in advance in accordance with the rules in the remarks column of Fig. 10. In the example of Fig. 10, the scores are set to a plurality of levels of numbers, and the larger the number, the higher the score.

The score table 248 includes "low warpage" and a "difference between physical property values" as the demand characteristics. The score of each of the "low warpage" and the "difference between physical property values" changes depending on the shape value and the physical property value received by the reception unit 232. Specifically, the score of the "low warpage" increases for individual materials as the amount of warpage simulated by the simulation unit 34 decreases using the shape value received by the reception unit 232 and the physical property values of the materials registered in the material DB 40. The score of the "difference between physical property values" increases as the difference between physical property values calculated by the calculation unit 36, that is, the difference between the shape value received by the reception unit 232 and the physical property values of the materials registered in the material DB 40 decreases.

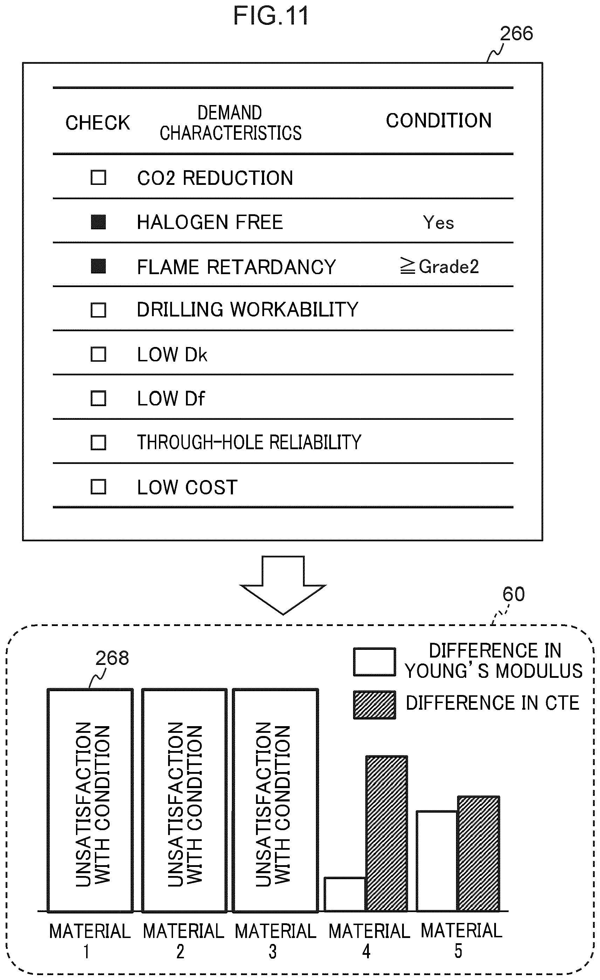

An example of a filter designation screen 266 is illustrated in the upper diagram of Fig. 11. Of the demand characteristics included in the score table 248, the filter designation screen 266 lists demand characteristics other than the "low warpage" and the "difference between physical property values", and associates the "check" column and the "condition" column with the individual demand characteristics. In the "check" column, a check box for selecting a demand characteristic which designates a filtering condition is displayed. The "condition" column is a column for inputting a condition for a demand characteristic checked in the "check" column. The "condition" column may be, for example, an input column in which a condition that can be designated in the corresponding demand characteristic can be selected in a pull-down menu or the like. The reception unit 232 receives, via the filter designation screen 266, selection of the demand characteristic that designates a filtering condition and a filtering condition for the selected demand characteristic.

When the score button 264 is selected on the display screen 250, of the demand characteristics, the reception unit 232 receives selection of a target demand characteristic which is reflected in a combined score calculated by the scoring unit 247 to be described below. The reception unit 232 receives a specific weighting when scores for the selected demand characteristic are combined into the combined score.

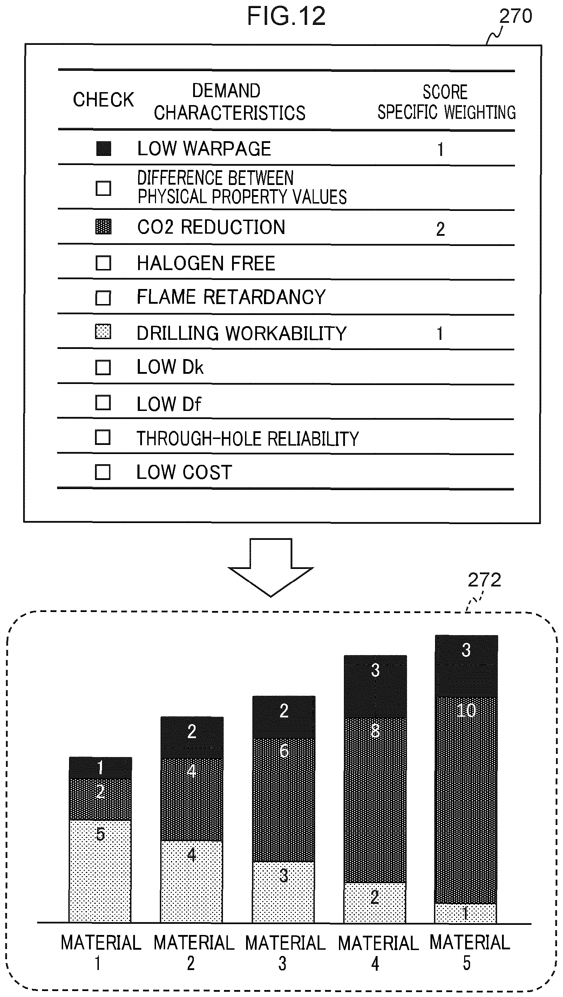

Specifically, the reception unit 232 displays a score designation screen for receiving the selection of the target demand characteristic and a score specific weighting with regard to each of the plurality of demand characteristics defined in the score table 248. An example of a score designation screen 270 is illustrated in the upper diagram of Fig. 12. The score designation screen 270 lists the demand characteristics included in the score table 248, and associates the "check" column and the "score specific weighting" column with individual demand characteristics. In the "check" column, a check box for selecting a demand characteristic for reflecting the score in the combined score is displayed. The "score specific weighting" column is a column for inputting the specific weighting when the score with respect to the demand characteristic checked in the "check" column is reflected in the combined score. The reception unit 232 receives the selection of the target demand characteristic and the score specific weighting via the score designation screen 270.

The filter unit 246 performs filtering of each of the plurality of materials registered in the material DB 40 on the basis of whether or not the condition received by the reception unit 232 is satisfied. Specifically, the filter unit 246 converts the designated condition into a score by using a predetermined conversion expression for the designated demand characteristic, compares the score with the scores of the materials defined in the score table 248, and determines whether or not the condition is satisfied.

For example, in a case in which a condition of "Yes" is designated for the demand characteristic "halogen free", the filter unit 246 determines that the materials 1 and 2 are not suitable and the materials 3, 4, and 5 are suitable when the score table 248 as illustrated in Fig. 10 is used. For example, in a case in which a condition of "≥ Grade 2" is designated for the demand characteristic "flame retardancy", the filter unit 246 determines that a material given a score equal to or higher than a score corresponding to Grade 2 (for example, 2) is suitable.

The conditions to be designated for the demand characteristics may be options that are easy for the user to understand, such as "Yes or No" or "Grade 1, Grade 2, ···". A correspondence relationship between the scores defined in the score table 248 and the options of the conditions may be set as the conversion expression described above. The correspondence relationship between the scores and the options of the conditions is not limited to the case of being set by the conversion expression, and may be set by a table or the like indicating the correspondence relationship.

The scoring unit 247 combines the scores for the demand characteristics received by the reception unit 232 with regard to each of the plurality of materials and calculates a combined score. For example, the scoring unit 247 calculates, as the combined score with regard to each material, a weighted sum of scores of the demand characteristics according to the designated score specific weighting.

The display control unit 238 performs display control of the display screen 250 similarly to the display control unit 38 according to the first embodiment.

The display control unit 238 displays a filtering result obtained by the filter unit 246 in association with the display of the difference between the physical property values with regard to each of the plurality of materials. For example, as illustrated in the lower diagram of Fig. 11, the display control unit 238 superimposes and displays a mask 268 on the graph for the material determined to be a material which does not satisfy the condition, of the graphs indicating the differences between the physical property values for the respective materials displayed in the physical property value difference display region 60. A character, a mark, or the like indicating that the conditions are not satisfied may be displayed on the mask 268. The display control unit 238 is not limited to the case of displaying the mask 268, and may display a character, a mark, or the like indicating that the material does not satisfy the condition on the graph of the corresponding material, or may delete the graph of the material that does not satisfy the condition from the physical property value difference display region 60.

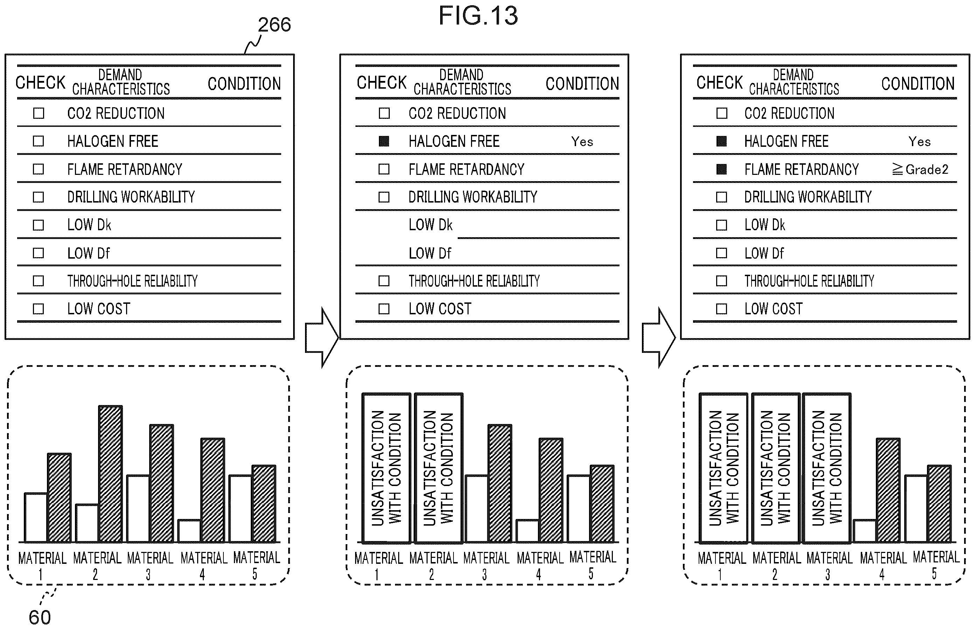

As illustrated in Fig. 13, the display control unit 238 updates the display of the physical property value difference display region 60 each time the reception unit 232 receives a filtering condition via the filter designation screen 266. In the example of Fig. 13, the left diagram illustrates a state in which the filtering condition is not designated, and the center diagram and the right diagram illustrate states in which the filtering condition is designated. In the center diagram, a condition is designated for one demand characteristic ("halogen free"), and in the right diagram, a condition is designated for another demand characteristic ("flame retardancy"), so that materials which do not satisfy the condition increase in order from the left.

The display control unit 238 performs control to display the combined score calculated by the scoring unit 247 with regard to each of the plurality of materials. For example, the display control unit 238 displays a score screen 272 as illustrated in the lower diagram of Fig. 12. The score screen 272 may be superimposed on the display screen 250 and displayed in another window, may be displayed on the display screen 250 instead of the physical property value difference display region 60, or may be displayed together with the physical property value difference display region 60 by changing the arrangement of the display screen 250. In the example of Fig. 12, the combined score of each material is represented by a bar graph, and details of the scores for the respective demand characteristics contained in the combined score are represented by color coding (shading) of the bar graph and numbers in the graph. As illustrated in Fig. 12, the color coding of the bar graph indicating the details of the score may be associated with the color coding of the "check" column on the score designation screen 270.

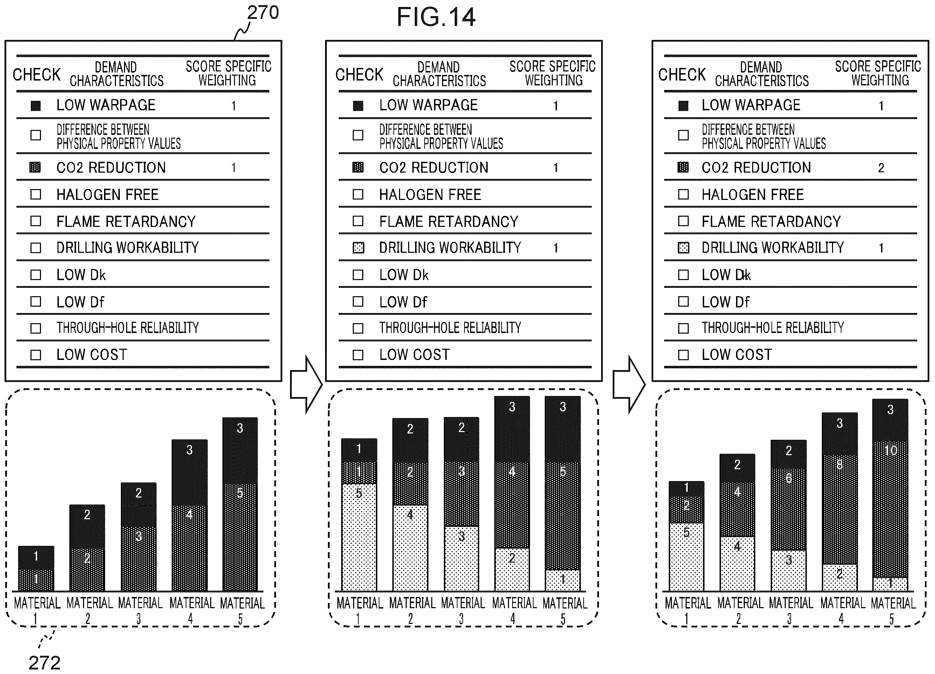

As illustrated in Fig. 14, the display control unit 238 updates the display of the score screen 272 each time the reception unit 232 receives the designation of score calculation via the score designation screen 270. In the example of Fig. 14, in the left diagram, both the "low warpage" and the "CO2 reduction" are selected with the score specific weighting of 1, and in the center diagram, the selection of "drilling workability" is further added with the score specific weighting of 1. In the right diagram, the score specific weighting of "CO2 reduction" is changed to 2.

Next, operations of the material selection assistance apparatus 210 according to the second embodiment will be described.

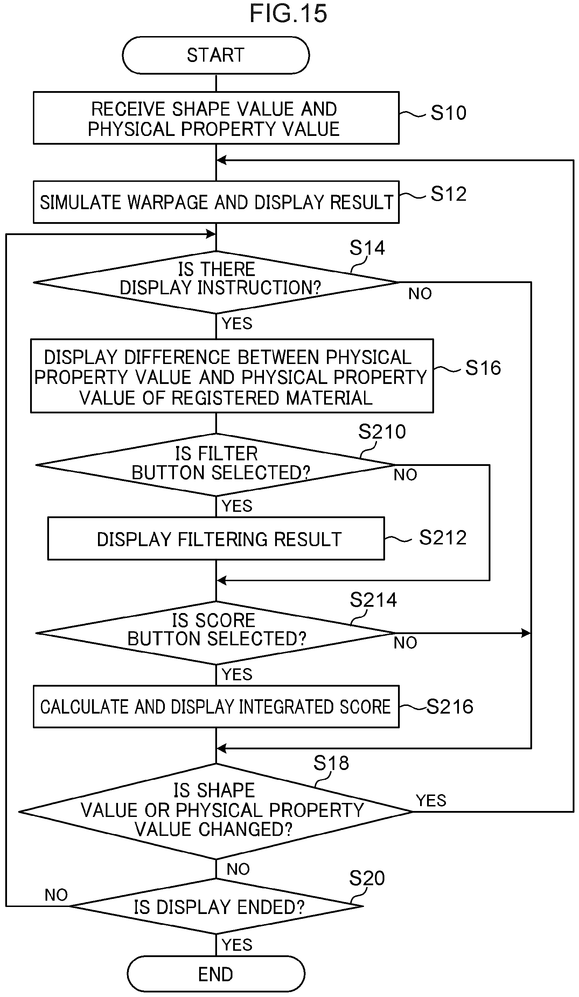

Fig. 15 is a flowchart illustrating a flow of a material selection assistance process executed by the CPU 12 of the material selection assistance apparatus 210. When the CPU 12 reads the material selection assistance program from the storage device 16, loads the program in the memory 14, and executes the program, the CPU 12 functions as the functional configurations of the material selection assistance apparatus 210, and the material selection assistance process illustrated in Fig. 15 is executed. In the material selection assistance process according to the second embodiment, the same step numbers are assigned to the same processes as the material selection assistance process according to the first embodiment, and the detailed description thereof will be omitted.

In the next step S210, the reception unit 232 determines whether or not the filter button 262 on the display screen 250 is selected through steps S10 to S16. The process proceeds to step S212 in the case in which the filter button 262 is selected, and the process proceeds to step S214 in the case in which the filter button 262 is not selected.

In step S212, the reception unit 232 displays the filter designation screen 266 and receives designation of a filter condition. The filter unit 246 converts the designated condition into a score by using a predetermined conversion expression for the designated demand characteristic, compares the score with the scores of the materials defined in the score table 248, and determines whether or not the condition is satisfied. The display control unit 238 performs superimposition, displaying, and the like of the mask 268 on the graph for the material determined to be a material which does not satisfy the condition, of the graphs indicating the differences between the physical property values for the respective materials displayed in the physical property value difference display region 60, and displays a filtering result.

Next, in step S212, the reception unit 232 determines whether or not the score button 264 of the display screen 250 is selected. The process proceeds to step S216 in the case in which the score button 264 is selected, and the process proceeds to step S 18 in the case in which the score button 264 is not selected.

In step S216, the reception unit 232 displays the score designation screen 270, and receives the selection of the demand characteristics and the score specific weighting which are reflected in the combined score. The scoring unit 247 calculates, as the combined score with regard to each material, a weighted sum of scores of the demand characteristics according to the designated score specific weighting. The display control unit 238 displays the score screen 272 in which the combined score of each material is represented by a bar graph. Then, steps S 18 and S20 are executed similarly to those in the first embodiment.

As described above, the material selection assistance apparatus according to the second embodiment executes, for the materials, the filtering process in which the condition for the demand characteristic other than the warpage of the substrate is designated for the display of the difference between the physical property value and the designated physical property value. This enables whether or not other demand characteristic conditions are satisfied to be taken into consideration, in addition to the simulation result of the warpage and the difference between physical property values, and enables the material selection to be more easily performed.

The material selection assistance apparatus according to the second embodiment displays, with regard to each material, a score for a designated demand characteristic of the demand characteristics including the simulation result of the warpage and the difference between physical property values. This enables the material selection to be more easily performed in the comprehensive consideration of demand characteristics.

### <Modification Example 1>

In the embodiments described above, the case in which the warpage of the semiconductor package substrate is simulated as a phenomenon occurring in the product has been described, but the disclosure is not limited thereto. In Modification Example 1, the service life of the solder in the semiconductor package substrate is simulated. In this case, the configuration of the semiconductor package includes the solder in addition to the examples of the configurations of the embodiments described above. The physical property value includes a material constant of the solder calculated by the Coffin-Manson law.

In Modification Example 1, the simulation unit simulates, by thermal stress analysis, the strain generated in the solder according to a temperature cycle test, which is a method of evaluating the bonding reliability of the solder. Examples of simulation conditions include an initial temperature, a temperature amplitude (a maximum value and a minimum value of the temperature), maintenance times of the maximum temperature and the minimum temperature, a temperature lowering rate and a temperature raising rate, and the total number of cycles to be applied. The simulation unit simulates the amount of increase in plastic strain per one temperature cycle and the amount of plastic strain that increases in one temperature cycle, and calculates the service life of the solder with any material constant by a model expression based on the Coffin-Manson law. Similarly to the embodiments described above, in the simulation, a function created in advance may be used.

The inputs of the shape value and the physical property value, the display of the simulation result, the calculation and the display of the difference between the physical property values, and the like in Modification Example 1 are the same as those in the embodiments described above.

The stress of the semiconductor package substrate may be simulated as the phenomenon occurring in the product. Also in this case, similarly to the embodiments described above, the inputs of the shape value and the physical property value may be received, the stress may be simulated using, for example, a function created in advance, and the display of the simulation result, the calculation and the display of the difference between physical property values, and the like may be performed.

### <Modification Example 2>

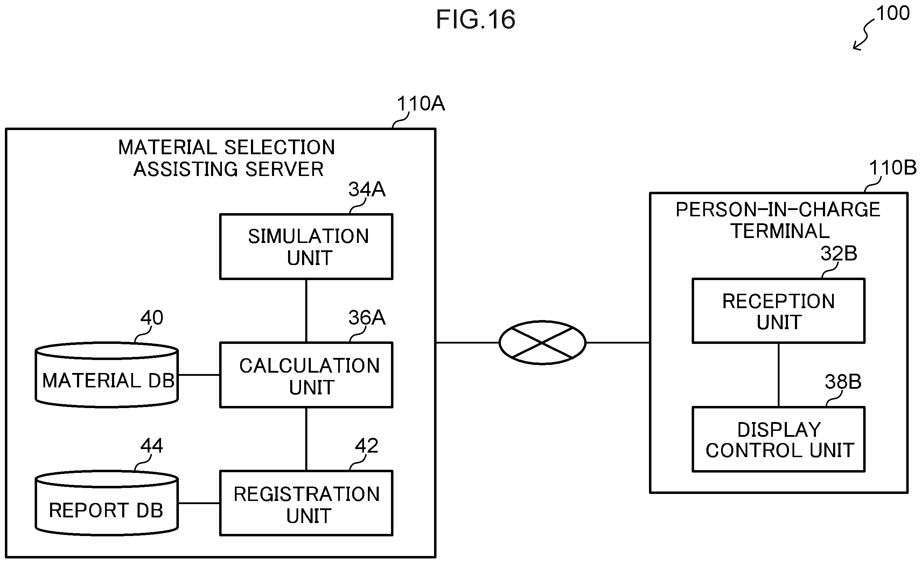

In both the embodiments described above, the configurations in which the material selection assistance apparatus functions as a single apparatus has been described, but in Modification Example 2, as illustrated in Fig. 16, a material selection assistance system 100 including a material selection assistance server 110A and a person-in-charge terminal 110B will be described. The material selection assistance server 110A and the person-in-charge terminal 110B are connected via a network. The number of each of the material selection assistance servers 110A and the person-in-charge terminals 110B included in the material selection assistance system 100 is not limited to the example of Fig. 16. In Modification Example 2, reference numerals having common numerical part are assigned to configurations common to those of the first embodiment, and differences from the first embodiment will be mainly described, and detailed description of common functions will be omitted.

The material selection assistance server 110A functionally includes a simulation unit 34A, a calculation unit 36A, and a registration unit 42. A material DB 40 and a report DB 44 are stored in a predetermined storage area of the material selection assistance server 110A.

The simulation unit 34A receives a shape value and a physical property value input via the display screen 50 from the person-in-charge terminal 110B. The simulation unit 34A delivers the received physical property value to the calculation unit 36A. The simulation unit 34A transmits a simulation result to the person-in-charge terminal 110B. The calculation unit 3 6A transmits a calculation result of a difference between the physical property value of each material stored in the material DB 40 and the input physical property value to the person-in-charge terminal 110B. The registration unit 42 receives a report (details thereof will be described below) from the person-in-charge terminal 110B, and registers the received report in the report DB 44.

The person-in-charge terminal 110B is an information processing terminal such as a personal computer, a tablet terminal, or a smartphone held by the person in charge. The person-in-charge terminal 110B functionally includes a reception unit 32B and a display control unit 38B.

The reception unit 32B transmits the shape value and the physical property value input via the display screen 50 to the material selection assistance server 110A. The reception unit 32B receives a customer ID which is the identification information of the customer, and creates a report associated with the customer ID when the display of the display screen 50 is ended. The reception unit 32B may input a final shape value and a final physical property value, the simulation result, and the difference between physical property values for each material in the report. In a case in which any material is selected from a plurality of materials, the reception unit 32B may receive information of the selected material and input the information in the report. The reception unit 32B may receive various items of information such as a customer's request and input the information in the report. The reception unit 32B transmits the created report to the material selection assistance server 110A.

According to Modification Example 2, a plurality of people in charge can use a service provided by the material selection assistance system 100 via the respective person-in-charge terminals 110B, and processing in each person-in-charge terminal 110B can be reduced. The report stored in the report DB 44 of the material selection assistance server 110A can be shared by the people in charge and can be effectively used.

In Modification Example 2, the case in which the configurations of the material selection assistance apparatus 10 according to the first embodiment are changed to the configurations of the material selection assistance system 100 including the material selection assistance server 110A and the person-in-charge terminal 110B has been described. However, this is similarly applicable to the second embodiment. Specifically, the material selection assistance server side may include a filter unit 246A, a scoring unit 247A, and a score table 248, and the reception unit 32B and the display control unit 38B of the person-in-charge terminal 110B may be a reception unit 232B and a display control unit 238B.

The material selection assistance process executed by the CPU reading software (program) in both the embodiments described above may be executed by various processors other than the CPU. Examples of the processors in this case include a programmable logic device (PLD) in which a circuit configuration can be changed after manufacture, such as a field-programmable gate array (FPGA), a dedicated electric circuit which is a processor having a circuit configuration based on dedicated design for executing specific processing, such as an application specific integrated circuit (ASIC), and the like. The material selection assistance process may be executed by one of these various processors, or may be executed by a combination of two or more processors of the same type or different types (for example, a combination of a plurality of FPGAs and a combination of a CPU and an FPGA). More specifically, a hardware structure of these various processors is an electric circuit in which circuit elements such as semiconductor elements are combined.

In both the embodiments described above, an aspect in which the material selection assistance program is stored (installed) in the storage device in advance has been described, but the disclosure is not limited thereto. The program may be provided in a state of being stored in a storage medium such as a CD-ROM, a DVD-ROM, or a USB memory. The program may be provided in a state of being downloaded from an external device via a network.

### REFERENCE SIGNS LIST

10, 210 material selection assistance apparatus
12 CPU
14 Memory
16 Storage device
18 Input device
20 Output device
22 Storage medium reading device
24 Communication I/F
26 Bus
32, 32B, 232, 232B Reception unit
34, 34A Simulation unit
3636A Calculation unit
38, 38B, 238, 238B Display control unit
40 Material DB
42 Registration unit
44 Report DB
246, 246A Filter unit
247, 247A Scoring unit
248 Score table
50, 250 Display screen
52 Shape value input region
52A Arrangement designation region
52B Dimension input region
54 Physical property value input region
56 Display instruction button
58 Simulation result display region
60 Physical property value difference display region
262 Filter button
264 Score button
266 Filter designation screen
268 Mask
270 Score designation screen
272 Score screen
100 material selection assistance system
110A material selection assistance server
110B Person-in-charge terminal

## Claims

1. A material selection assistance apparatus, comprising:
a reception unit that receives a shape value pertaining to a shape of a product and a physical property value of a material used for the product;
a simulation unit that simulates a phenomenon occurring in the product based on the shape value and the physical property value received, each time the shape value and the physical property value are received by the reception unit;
a calculation unit that calculates, with regard to each of a plurality of materials registered in advance, a difference between a physical property value of each of the plurality of materials and the physical property value received by the reception unit; and
a display control unit that performs control to display a simulation result obtained by the simulation unit and the difference calculated by the calculation unit at a display device.

2. The material selection assistance apparatus according to claim 1, wherein the simulation unit simulates the phenomenon using a function created in advance to output a physical quantity indicating the phenomenon, with the shape value, the physical property value, and a simulation condition as inputs.

3. The material selection assistance apparatus according to claim 1 or 2, wherein the display control unit performs control to display, on one screen, an input region to which the shape value and the physical property value are input, a display region of the simulation result, and a display region of the difference with regard to each of the plurality of materials.

4. The material selection assistance apparatus according to claim 1 or 2, wherein the display control unit performs control to display the difference with regard to each of the plurality of materials in a graph.

5. The material selection assistance apparatus according to claim 1 or 2, wherein:
the reception unit receives a display instruction for the difference with regard to each of the plurality of materials, and
when the display instruction is received, the calculation unit calculates a difference between the shape value and the physical property value received by the reception unit, and a physical property value of each of the plurality of materials.

6. The material selection assistance apparatus according to claim 1 or 2, wherein:
the reception unit receives a condition for one or more characteristics required of the product other than the phenomenon,
the material selection assistance apparatus comprises a filter unit that performs filtering of each of the plurality of materials based on whether or not the condition received by the reception unit is satisfied, and
the display control unit performs control to display a filtering result obtained by the filter unit at the display device in association with the display of the difference with regard to each of the plurality of materials.

7. The material selection assistance apparatus according to claim 6, wherein the display control unit superimposes and displays a mask indicating that the condition is not satisfied on a graph for a material determined by the filter unit to be a material which does not satisfy the condition, among graphs respectively indicating the differences for the plurality of materials.

8. The material selection assistance apparatus according to claim 1 or 2, further comprising:
a scoring unit that calculates, with regard to each of the plurality of materials, a combined score obtained by combining a score corresponding to the simulation result obtained by the simulation unit, a score corresponding to the difference, and a score determined in advance with regard to each of one or more characteristics required of the product other than the phenomenon and the difference,
wherein the display control unit performs control to display the combined score calculated by the scoring unit at the display device, with regard to each of the plurality of materials.

9. The material selection assistance apparatus according to claim 8, wherein:
the reception unit receives a selection of a target from among the simulation result, the difference, and the one or more characteristics reflected in the combined score, and a specific weighting when scores for the selected target are combined into the combined score, and
the scoring unit calculates the combined score based on the target and the specific weighting received by the reception unit.

10. The material selection assistance apparatus according to claim 9, wherein the display control unit displays details of a score with regard to each of characteristics of the combined score.

11. The material selection assistance apparatus according to claim 1 or 2, wherein, in a case in which the product is a semiconductor package, the phenomenon is warpage of the product, stress of the product, or a service life of solder in the product.

12. A material selection assistance method, comprising:
receiving, by a reception unit, a shape value pertaining to a shape of a product and a physical property value of a material used for the product;
simulating, by a simulation unit, a phenomenon occurring in the product, based on the shape value and the physical property value received, each time the shape value and the physical property value are received by the reception unit;
calculating, by a calculation unit, with regard to each of a plurality of materials registered in advance, a difference between a physical property value of each of the plurality of materials and the physical property value received by the reception unit; and
performing control, by a display control unit, to display a simulation result obtained by the simulation unit and the difference calculated by the calculation unit at a display device.

13. A material selection assistance program for causing a computer to function as:
a reception unit that receives a shape value pertaining to a shape of a product and a physical property value of a material used for the product;
a simulation unit that simulates a phenomenon occurring in the product based on the shape value and the physical property value received, each time the shape value and the physical property value are received by the reception unit;
a calculation unit that calculates, with regard to each of a plurality of materials registered in advance, a difference between a physical property value of each of the plurality of materials and the physical property value received by the reception unit; and
a display control unit that performs control to display a simulation result obtained by the simulation unit and the difference calculated by the calculation unit at a display device.
